# EUROPÄISCHE PATENTSCHRIFT

(11) **EP 1 017 277 B1**
(45) Veröffentlichungstag und Bekanntmachung des Hinweises auf die Patenterteilung: **24.04.2002**
(21) Anmeldenummer: 98950040.0
(22) Anmeldetag: 16.09.1998
(51) Int. Cl.: A01N 35/02, A61L 2/18, C11D 3/48

(54) **VERFAHREN ZUR REINIGUNG UND DESINFEKTION VON MEDIZINISCHEN INSTRUMENTEN**
METHOD FOR CLEANING AND DISINFECTING MEDICAL INSTRUMENTS
PROCEDE POUR LE NETTOYAGE ET LA DESINFECTION D'INSTRUMENTS MEDICAUX

(30) Priorität: 25.09.1997 DE 19741910
(43) Veröffentlichungstag der Anmeldung: 12.07.2000
(73) Patentinhaber: HENKEL-ECOLAB GmbH & CO. OHG, 40589 Düsseldorf (DE)
(72) Erfinder: DISCH, Karlheinz, D-42781 Haan (DE); BANSEMIR, Klaus-Peter, D-40764 Langenfeld (DE)
(74) Vertreter: Mathes, Nikolaus, Dr.
(86) Internationale Anmeldenummer: EP9805877
(87) Internationale Veröffentlichungsnummer: WO9915012

(56) Entgegenhaltungen:
- DE-A- 19 603 977
- US-A- 5 480 643

## Beschreibung

Die vorliegende Erfindung liegt auf dem Gebiet der Desinfektion von medizinischen Geräten und Instrumenten und betrifft ein Verfahren zur insbesondere manuellen Aufbereitung derartiger Geräte und Instrumente.

Zur Reinigung und Desinfektion von medizinischen Instrumenten kennt man verschiedene Verfahren, in denen Reinigungs- und Desinfektionsvorgang getrennt oder mehr oder weniger stark kombiniert ablaufen. Die Einzelschritte können manuell oder aber mit Hilfe geeigneter Reinigungs- bzw. Desinfektionsmaschinen durchgeführt werden, wobei in der maschinellen Aufbereitung im allgemeinen höhere Temperaturen angewandt werden können mit der Folge, daß Reinigung und/oder Desinfektion bei diesen Verfahren schneller ablaufen. Bei den manuellen Aufbereitungsverfahren, die im allgemeinen bei Raumtemperatur oder nur wenig erhöhter Temperatur ablaufen, kommt der Zusammensetzung der Reinigungs- bzw. Desinfektionslösung, mit der die medizinischen Instrumente und Geräte behandelt werden, besondere Bedeutung zu.

Während bei maschinellen Reinigungs- und Desinfektionsverfahren, wie sie beispielsweise in der deutschen Patentanmeldung DE 38 16 734 beschrieben werden, mit wäßrigen Lösungen von nichtionogenem Tensid, proteolytischem Enzym, Komplexbildner und Aldehyd als mikrobizidem Wirkstoff bei Temperaturen zwischen 55 und 65 °C in kurzen Einwirkungszeiten hervorragende Ergebnisse erzielt werden, ist die Reinigungsleistung der dort eingesetzten Reinigungsflotten bei Zimmertemperatur in vielen Fällen, insbesondere bei hoher Blutbelastung oder hart angetrockneten Blut- und Eiweißresten nicht ausreichend. Ein Grund dafür wird in der Wechselwirkung zwischen Aldehyden und Eiweißkörpem gesehen, die zur Eiweißkoalugation führt und damit die Eiweißverschmutzungen bei Raumtemperatur sehr schwer entfernbar werden läßt. Da man andererseits auf die hervorragende Desinfektionswirkung der Aldehyde vielfach nicht verzichten möchte, ist vorgeschlagen vorden, diesen Lösungen zur manuellen Instrumentendesinfektion sogenannte Reinigungsverstärker zuzusetzen. Diese Reinigungsverstärker enthalten Alkalien, insbesondere Alkalicarbonat oder Alkalihydroxid, und verleihen der aldehydhaltigen Reinigungs- und Desinfektionslösung eine bessere Reinigungsleistung und eine stärkere Desinfektionswirkung gegenüber bestimmten Keimen. Zur Herstellung derartiger Reinigungs- und Desinfektionslösungen werden konzentrierte Aldehydlösungen und Reinigungsverstärker als Kombination beispielsweise in der Patentanmeldung WO 91/16083 vorgeschlagen. Nachteilig ist, daß durch den Zusatz dieser Art von Reinigungsverstärkern die Gebrauchsdauer der Reinigungs- und Desinfektionslösung deutlich herabgesetzt wird, da die Aldehyde in der Regel in alkalischer Lösung nicht beständig sind und deshalb die Desinfektionswirkung entscheidend nachläßt. Eine andere Alternative besteht darin, mit getrennten Lösungen zu arbeiten, wobei zunächst gereinigt und dann mit einer zweiten Lösung, die Aldehyd enthält, desinfiziert wird. Abgesehen vom Arbeitsaufwand ist diese Verfahrensweise aus arbeitshygienischer Sicht wegen der Infektionsgefahr in der ersten Stufe wenig praxisgerecht.

Die US-A-5,480,643 beschreibt eine stabile, feste oder halbfeste antimikrobielle Zusammensetzungen, welche neben Dialdehyden und Kohlehydraten auch Puffersubstanzen wie z.B. Monoethanolamin, Diethanolamin und Triethanolamin enthalten können.

Aufgabe der vorliegenden Erfindung war es in diesem Zusammenhang ein verbessertes Verfahren für die manuelle Reinigung und Desinfektion von medizinischen Instrumenten unter Verwendung von wäßrigen Aldehydlösung bereitzustellen.

Gegenstand der vorliegenden Erfindung ist ein Verfahren zur Reinigung und Desinfektion von medizinischen Instrumenten, bei dem die zu behandelnden Instrumente in einer ersten Stufe mit einer wäßrigen Reinigungs- und Desinfektionslösung in Kontakt gebracht werden, die mindestens einen Aldehyd aus der Gruppe der Mono- und Dialdehyde mit 1 bis 8 C-Atomen und mindestens ein Tensid sowie gegebenenfalls weitere Hilfs- und Zusatzstoffe enthält und einen pH-Wert im schwach alkalischen Bereich aufweist, in einer zweiten Stufe mit Wasser gespült und dann getrocknet werden und das dadurch gekennzeichnet ist, daß die Reinigungs- und Desinfektionslösung ein Alkanolamin aus der Gruppe Monoethanolamin, Diethanolamin, Triethanolamin und deren Mischungen enthält. Vorzugsweise enthält die Reinigungs- und Desinfektionslösung in diesem Verfahren Diethanolamin.

in diesem neuen Verfahren werden Blut- und Eiweißanschmutzungen schnell und sicher von den Instrumenten entfernt und gleichzeitig eine hohe desinfizierende Wirkung selbst gegenüber schwer zu bekämpfenden Viren erreicht. Die in diesem Verfahren verwendeten Reinigungs- und Desinfektionslösungen besitzen eine ausgezeichnete Beständigkeit, so daß die einmal angesetzten Lösungen über mehrere Tage ohne Wirkungseinbuße verwendet werden können.

Bei den Aldehyden, die in den Reinigungs- und Desinfektionslösungen des erfindungsgemäßen Verfahrens eingesetzt werden, handelt es sich um Monound/oder Dialdehyde mit 1 bis 8 C-Atomen. Beispiele geeigneter Aldehyde sind Formaldehyd, Glyoxal, Succindialdehyd, Glutardialdehyd und Phthalaldehyd. Besonders bevorzugt werden Formaldehyd und die aliphatischen Dialdehyde mit 2 bis 6 C-Atomen im Molekül. Die Aldehyde liegen in der Desinfektionslösung vorzugsweise in freier Form vor, können aber auch teilweise oder vollständig in leicht hydrolisierbar gebundener Form, insbesondere in acetalisch gebundener Form vorliegen. Die Konzentration an Aldehyd insgesamt, gerechnet als freier Aldehyd, soll in den Reinigungs- und Desinfektionslösungen, die im Verfahren angewendet werden, vorzugsweise zwischen etwa 0,01 und etwa 3 Gew.-%, insbesondere zwischen etwa 0,05 und etwa 0,8 Gew.-% liegen.

In der Regel enthalten die im erfindungsgemäßen Verfahren verwendeten Reinigungs- und Desinfektionslösungen keine weiteren mikrobizid wirksamen Substanzen. In Einzelfällen kann es aber, etwa zur Abtötung besonderer Problemkeime, zweckmäßig sein, weitere mikrobizide Wirkstoffe, beispielsweise quartäre Ammoniumverbindungen, aliphatische und/oder aromatische Alkohole, Guanidinderivate und/oder Phenole zusätzlich zu den Aldehyden zu verwenden. Niedere einwertige Alkohole werden in den Lösungen aber allenfalls in geringer nicht mikrobizid wirksamer Konzentration, vorzugsweise mit weniger als 2 Gew.-%, insbesondere mit weniger als 1 Gew.% verwendet.

Als zweiten wesentlichen Wirkstoff enthalten die im erfindungsgemäßen Verfahren verwendeten Reinigungs- und Desinfektionslösungen Tensid, das in erster Linie die Aufgabe hat, für die Benetzung der Instrumente und die Ablösung von anhaftenden Verunreinigungen zu sorgen. Geeignet sind prinzipiell alle für derartige Reinigungsvorgänge brauchbaren Tenside aus den Klassen der nichtionischen, anionischen und zwitterionischen Tenside, soweit bei der Auswahl darauf geachtet wird, daß keine störenden Wechselwirkungen mit den Desinfektionswirkstoffen oder zwischen mehreren Tensiden unterschiedlicher Typen auftreten. Im allgemeinen werden schaumarme Tenside bevorzugt. Ein weiterer Vorzug gilt nichtionischen Tensiden wegen ihrer hohen Reinigungskraft gegenüber fettigen Verunreinigungen. Selbstverständlich können aber auch Mischungen mehrerer Tenside verwendet werden. Die Konzentration an Tensid insgesamt soll in den Reinigungs- und Desinfektionslösungen, die im Verfahren angewendet werden, vorzugsweise zwischen etwa 0,01 und etwa 2 Gew.-%, insbesondere zwischen etwa 0,05 und etwa 0,5 Gew.-% liegen.

Als nichtionische Tenside eignen sich in erster Linie die Anlagerungsprodukte von 3 bis 30 Mol Ethylenoxid (EO) an primäre C₁₀ - C₂₀-Alkohole, wie z.B. an Kokos- oder Talgfettalkohole, an Oleylalkohol, an Oxoalkohole oder an sekundäre Alkohole dieser Kettenlänge. Dabei können neben den hierbei umfaßten wasserlöslichen nichtionischen Tensiden auch die nicht vollständig wasserlöslichen niedrig ethoxylierten Fettalkohol-Polyglykolether mit 3 bis 7 Ethylenglykolethergruppen im Molekül von Interesse sein, vor allem dann, wenn sie zusammen mit wasserlöslichen nichtionischen oder anionischen Tensiden eingesetzt werden. Ebenfalls geeignet sind die entsprechenden Ethoxylierungsprodukte anderer langkettiger Verbindungen, beispielsweise der Fettsäuren und der Fettsäureamide mit 12 bis 18 C-Atomen und der Alkylphenole mit 8 bis 16 C-Atomen im Alkylteil. In all diesen Produkten kann anstelle eines Teils des Ethylenoxids auch Propylenoxid (PO) angelagert sein. Weitere geeignete nichtionische Tenside sind auch die wasserlöslichen, 20 bis 250 Ethylenglykolethergruppen und 10 bis 100 Propylenglykolethergruppen enthaltenden Anlagerungsprodukte von Ethylenoxid an Polypropylenglykol, Alkylendiaminpolypropyienglykol und an Alkylpolypropylenglykol mit 1 bis 10 Kohlenstoffatomen in der Alkylkette, in denen die Polypropylenglykolkette als hydrophober Rest fungiert. In den als nichtionische Tenside geeigneten Alkoxylierungsprodukten können die freien Hydroxylgruppen auch mit kurzkettigen Alkoholen mit vorzugsweise 1 bis 4 C-Atomen veräthert sein.

Als nichtionische Tenside eignen sich ebenfalls Mono- und Diethanolamide der Fettsäuren sowie langkettige Aminoxide oder Sulfoxide, beispielsweise die Verbindungen N-Kokoalkyl-N,N-dimethylaminoxid, N-Talgalkyl-N,N-dihydroxyethylaminoxid, und auch die wasserlöslichen Alkylglycoside, deren hydrophober C₈ - C₂₀-Alkylrest mit einem meist oligomeren hydrophilen Glycosidrest glykosidisch verknüpft ist, beispielsweise C₁₂ - C₁₄-Fettalkohol + 1,6 Glucose. In den erfindungsgemäß verwendeten Mitteln werden als nichtionische Tenside die Alkoxylate von Fettalkoholen oder Oxoalkoholen mit 5 bis 15 Mol EO und gegebenenfalls zusätzlich 1 bis 3 Mol PO sowie die mit C₁ - C₄-Alkoholen an der endständigen Hydroxylgruppe veretherten Alkoxylate dieses Typs bevorzugt.

Bei den synthetischen anionischen Tensiden, die in der Reinigungs- und Desinfektionslösung enthalten sein können, handelt es sich vor allem um solche vom Typ der Sulfonate und Sulfate. Als Tenside vom Sulfonattyp kommen Alkylbenzolsulfonate mit einem C₉ - C₁₅-Alkylrest und Olefinsulfonate, d.h. Gemische aus Alken- und Hydroxyalkansulfonaten sowie Disulfonaten, wie man sie beispielsweise aus C₁₂ - C₁₈-Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sich auch die Alkansulfonate, die aus C₁₂ - C₁₈-Alkanen durch Sulfochlorierung oder Sulfoxidation und anschließende Hydrolyse bzw. Neutralisation oder durch Bisulfitaddition an Olefine erhältlich sind, sowie die Ester von α-sulfonierten Methyl- oder Ethylester der hydrierten Kokos-, Palmkern- oder Talgfettsäuren.

Geeignete Tenside vom Sulfattyp sind die Schwefelsäuremonoester von langkettigen primären Alkoholen natürlichen oder synthetischen Ursprungs, d.h. von Fettalkoholen, wie z.B. Kokosfettalkoholen, Oleylalkohol, Lauryl-, Myristyl-, Palmityl- oder Stearylalkohol, oder den C₁₀ - C₂₀-Oxoalkoholen oder sekundären Alkoholen dieser Kettenlänge. Auch die Schwefelsäuremonoester der mit 1 bis 6 Mol Ethylenoxid (EO) ethoxylierten aliphatischen langkettigen primären Alkohole bzw. ethoxylierten sekundären Alkohole sind geeignet. Ferner eignen sich sulfatierte Fettsäurealkanolamide, sulfatierte Fettsäuremonoglyceride, langkettige Sulfobernsteinsäureester sowie die Salze von langkettigen Ethercarbonsäuren, die beispielsweise durch Umsetzung langkettiger, mit 1 bis 10 Mol EO ethoxylierter Alkohole mit Chloressigsäure erhältlich sind. Die anionischen Tenside werden vorzugsweise als Alkalisalze, insbesondere Natriumsalze eingesetzt, doch können auch die Salze von Alkanolaminen mit 2 bis 6 C-Atomen verwendet werden. Besonders bevorzugte Aniontenside sind im Rahmen der vorliegenden Erfindung die Alkylbenzolsulfonate, die Alkansulfonate, die Olefinsulfonate und die Fettalkoholsulfate.

Anstelle von synthetischen anionischen und/oder nichtionischen Tensiden oder zusammen mit diesen können auch amphotere (zwitterionische) Tenside und insbesondere Seifen in größerer Menge enthalten sein. Bei den amphoteren Tensiden handelt es sich um langkettige Verbindungen, deren hydrophiler Teil aus einem kationisch geladenem Zentrum (üblicherweise eine tertiäre Aminooder eine quartäre Ammoniumgruppe) und einem anionisch geladenem Zentrum (üblicherweise eine Carboxylat- oder eine Sulfonatgruppe) besteht. Beispiele derartiger Tenside sind N-Kokosalkyl-N,N-dimethylaminoacetat und N-Dodecyl-N,N-dimethyl-3-aminopropansulfonat. Bei den Seifen handelt es sich um die Alkali- oder Ammoniumsalze der Fettsäuren mit 12 bis 18 C-Atomen in der Kette. Beispiele sind C₁₀ - C₁₈-Kokosfettsäurenatriumsalz, C₁₆ - C₁₈-Talgalkylhydroxyethylammoniumsalz und Myristinsäurekaliumsalz.

Als dritte wesentliche Komponente enthält die im erfindungsgemäßen Verfahren verwendete Reinigungs- und Desinfektionslösung mindestens ein Amin aus der Gruppe Ethanolamin, Diethanolamin und Triethanolamin. Vorzugsweise werden Diethanolamin oder Mischungen, die überwiegend Diethanolamin enthalten, verwendet. Der Zusatz dieser Amine zur Reinigungs- und Desinfektionslösung bewirkt eine ganz erhebliche Verbesserung bei der Ablösung von organischen Verunreinigungen von den Instrumenten, wobei in vielen Fällen eine noch bessere Reinigung als bei Zusatz von anorganischen Alkalien als Reinigungsverstärker zu beobachten ist. Gleichzeitig wird eine Verbesserung der Desinfektionswirkung gegenüber Sporen und hartnäckigen Viren beobachtet. Andererseits wird die Standzeit der aldehydischen Reinigungs- und Desinfektionslösungen praktisch nicht negativ durch den Zusatz dieser Amine beeinflußt. Die Menge an diesen Aminen beträgt in der Reinigungs- und Desinfektionslösung vorzugsweise zwischen etwa 0,01 und etwa 1 Gew.-%, insbesondere zwischen etwa 0,05 und etwa 0,3 Gew.-%. In jedem Falle soll die Lösung soviel an diesen Aminen enthalten, daß der pH-Wert der Lösung im schwach alkalischen Bereich vorzugsweise zwischen etwa 7 und etwa 10, insbesondere zwischen etwa 8 und etwa 9,5 liegt.

Neben diesen Bestandteilen kann die Reinigungs- und Desinfektionslösung des erfindungsgemäßen Verfahrens weitere Hilfs- und Zusatzstoffe enthalten, soweit dies für die Erreichung bestimmter Eigenschaften vorteilhaft ist. Zu diesen Hilfsund Zusatzstoffen zählen in erster Linie Komplexbildner für die Bestandteile der Wasserhärte, Farbstoffe, Duftstoffe, Korrosionsinhibitoren (beispielsweise Benzotriazol) sowie Konfektionierungshilfsmittel, wenn die Lösung aus vorgefertigten Konzentraten durch Verdünnung hergestellt wird. Die Menge an Hilfs- und Zusatzstoffen kann im Einzelfall sehr unterschiedlich sein und richtet sich nach der erwünschten Wirkung. Vorzugsweise enthält die Reinigungs- und Desinfektionslösung zwischen 0 und etwa 3 Gew.-% und insbesondere zwischen 0,1 und 2 Gew.-% an Hilfs- und Zusatzstoffen.

Die Komplexbildner werden vorzugsweise aus den Gruppen der Polycarbonsäuren, Hydroxypolycarbonsäuren, Phosphonocarbonsäuren, Polyphosphonsäuren, Aminopolyphosphonsäuren und Aminopolycarbonsäuren und deren wasserlöslichen Salzen, insbesondere den Alkalisalzen, ausgewählt. Beispiele derartiger Komplexbildner sind Nitrilotriessigsäure, Ethylendiamintetraessigsäure, Ethylendiamintetramethylenphosphonsäure, Hydroxyethan-1,1-diphosphonsäure, Phosphonobutantricarbonsäure, Weinsäure, Zitronensäure und Glukonsäure sowie deren Natriumsaize. Besonders bevorzugt werden im Rahmen des erfindungsgemäßen Verfahrens Nitrilotriessigsäure, Phosphonobutantricarbonsäure und deren Salze. Die Menge an Komplexbildner, gerechnet als freie Säure, liegt in den erfindungsgemäß verwendeten Reinigungs- und Desinfektionsmitteln vorzugsweise nicht über 0,5 Gew.-%, insbesondere zwischen etwa 0,02 und etwa 0,3 Gew.-%.

Da die Reinigungs- und Desinfektionslösungen, wie sie im Verfahren angewendet werden, in dieser Form nicht beliebig lange lagerfähig sind, werden sie im allgemeinen vor Gebrauch durch Auflösen der Bestandteile in Wasser hergestellt. Man geht dabei in der Regel aber nicht von einzelnen Bestandteilen, sondern von vorgefertigten Konzentraten aus, die jeweils für sich einen Teil der Komponenten in einer hochkonzentrierten, aber lagerfähigen Form enthalten. Diese Konzentrate können über die bereits genannten Bestandteile hinaus Konfektionierungshilfsmittel enthalten, die die Lagerstabilität erhöhen. Beispiele derartiger Konfektionierungshilfsmittel sind Lösungsvermittler und Konservierungsmittel.

Im allgemeinen geht man bei der Herstellung der Reinigungs- und Desinfektionslösung von zwei Konzentraten aus, von denen das eine schwach sauer eingestellt ist und die aldehydische Wirkstoffe enthält und das zweite alkalisch eingestellt ist und Tenside sowie die Ethanolamine enthält. Das die Aldehyde enthaltende Konzentrat kann darüber hinaus vor allem Komplexbildner in saurer Form, Tenside, Korrosionsinhibitoren und Alkohole als Lösungsvermittler enthalten, wobei mehrwertige Alkohole auch zur Dämpfung des Aldehydgeruchs eingesetzt werden. In dem alkalisch eingestellten Konzentrat können weiterhin vor allem Komplexbildner in Salzform und Lösungsvermittler enthalten sein.

Besonders bevorzugte Aldehyd enthaltende Konzentrate weisen folgende Zusammensetzung auf:
0,5 - 40 Gew.-%,vorzugsweise 10 - 30 Gew.-% Aldehyd,
0,5 - 20 Gew.-% vorzugsweise 1 - 10 Gew.-% Tensid
0,1 - 10 Gew.-%, vorzugsweise 0,5 - 5 Gew.-% Komplexbildner
zu 100 Gew.-% Hilfs- und Zusatzstoffe sowie Wasser

Besonders geeignete Reinigungsverstärkerkonzentrate weisen folgende Zusammensetzung auf:
1 - 20 Gew.-%, vorzugsweise 5 - 15 Gew.-% Alkanolamin
1 - 20 Gew.-%, vorzugsweise 5 - 15 Gew.-% Tensid
1 - 20 Gew.-%, vorzugsweise 5 - 15 Gew.-% Komplexbildner
zu 100 Gew.-% Hilfs- und Zusatzstoffe sowie Wasser.

Zur Herstellung der Reinigungs- und Desinfektionslösung kann im erfindungsgemäßen Verfahren Leitungswasser verwendet werden, wenn es eine ausreichende Reinheit aufweist. Auch zum Spülvorgang, der sich an die Reinigung und Desinfektion der Instrumente anschließt und der nach Abtrennung der Reinigungs- und Desinfektionslösung durchgeführt wird, kann Wasser dieser Qualität verwendet werden. Vorzugsweise wird aber mit sterilisiertem Wasser zumindest im letzten von mehreren Spülgängen gespült.

Die Einwirkungszeit der Reinigungs- und Desinfektionslösung richtet sich in erster Linie nach den zu bekämpfenden Keimen. Sie liegt vorzugsweise zwischen etwa 15 und etwa 60 Minuten bei Raumtemperatur. Bei höheren Temperaturen, beispielsweise bis etwa 40 °C und/oder bei gleichzeitiger Einwirkung von Ultraschall kann sie auch kürzer sein, bei Anwesenheit von Problemkeimen auch länger.

Anschließend werden die so behandelten Instrumente in geeigneter Weise getrocknet. lm einfachsten Falle kann dies durch Liegenlassen der Instrumente an der Luft geschehen. in der Regel wird das Trocknen aber durch Einlegen in Wärmeschränke oder durch Überblasen von Luft, die gegebenenfalls erwärmt sein kann, erreicht. Ggebenenfalls kann das Trocknen auch im Zusammenhang mit einem sich unmittelbar anschließenden Sterilisierungsprozeß vorgenommen werden.

### Beispiele

Zur Durchführung des erfindungsgemäßen Verfahrens wurden Reinigungs- und Desinfektionslösungen durch Verdünnen je eines Desinfektionskonzentrates und je eines Reinigungskonzentrates (Aktivator) mit Wasser hergestellt. Die verwendeten wäßrigen Desinfektionskonzentrate D1 und D2 hatten folgende Zusammensetzung:
**D1:**
   8,0 Gew.-% Glutaraldehyd
   4,0 Gew.-% Dimethyl(C_{12/14})alkylbenzylammoniumchiorid
   5,0 Gew.-% C_{12/14}-Fettalkohol + 7 EO
   25,0 Gew.-% alkoholische Lösungsmittel als Lösungsvermittler
   1,0 Gew.-% Natriumnitrilotriacetat
   1,0 Gew.-% Farbstoff, Parfüm, Korrosionsinhibitor
   57,0 Gew.-% Wasser
**D2:**
   11,1 Gew.-% Formaldehyd
   10,0 Gew.-% Glyoxal
   5,8 Gew.-% Glutaraldehyd
   5,0 Gew.-% C_{10/14}-Fettalkohol + 1,2 PO + 6,4 EO
   6,0 Gew.-% Ethanol
   0,5 Gew.-% Phosphonobutantricarbonsäure
   0,4 Gew.-% Natriumnitrilotriacetat
   0,9 Gew.-% Farbstoff, Parfüm, Korrosionsinhibitor
   60,3 Gew.-% Wasser

Die wäßrigen Reinigungskonzentrate R1 und R2 hatten folgende Zusammensetzung:
**R1:**
   8,0 Gew.-% C_{10/14}-Fettalkohol + 1,2 PO + 6,4 EO
   3,2 Gew.-% Natriumnitrilotriacetat
   8,0 Gew.-% Propandiol-1,2
   8,0 Gew.-% Monoethanolamin
   0,2 Gew.-% Parfüm und Farbstoff
   72,6 Gew.-% Wasser
**R2:**
   8,0 Gew.-% C_{10/14}-Fettalkohol + 1,2 PO + 6,4 EO
   3,2 Gew.-% Natriumnitrilotriacetat
   8,0 Gew.-% Propandiol-1,2
   8,0 Gew.-% Diethanolamin
   0,2 Gew.-% Parfüm und Farbstoff
   72,6 Gew.-% Wasser

Zum Vergleich wurde ein herkömmliches Aktivatorkonzentrat verwendet, das folgende Zusammensetzung hatte:
1,1 Gew.-% Natriumhydroxid
8,1 Gew.-% Natriumhydrogencarbonat
90,8 Gew.-% Wasser

### 1. Prüfung der Reinigungswirkung

Zur Prüfung der Reinigungswirkung im erfindungsgemäßen Verfahren wurden Edelstahlplättchen in standardisierter Weise mit Blut angeschmutzt, 1 Stunde lang in die Reinigungs- und Desinfektionslösung eingetaucht, nach dieser Zeit wieder entnommen und durch kurzes Schwenken in einem Wasserbad abgespült. Nach dem Trocknen an der Luft wurde die Menge an Blut, die auf den Plättchen nach dieser Reinigung noch vorhanden war, durch visuelle Abmusterung halbquantitativ ermittelt. Die Bewertung erfolgte in einer Skala von 1 bis 5, in der die Note 1 dann vergeben wurde, wenn keine Rückstände mehr sichtbar waren und die Note 5 ein Reinigungsergebnis bedeutete, wie es mit Wasser allein erzielt wurde. Die folgende Tabelle gibt die Reinigungsergebnisse im beschriebenen Verfahren bei Verwendung unterschiedlich zusammengesetzter Reinigungs- und Desinfektionslösungen an.

**Tabelle 1,**

| **Reinigungswirkung** | | |
|---|---|---|
| **Versuchs-Nr.** | **Zusammensetzung der Lösung** | **Reinigungsergebnis** |
| 1a | 2 Gew.-% D1 | 4 |
| 1b | 2 Gew.-% D1 + 2 Gew.-% R1 | 1 |
| 1c | 2 Gew.-% D1 + 2 Gew.-% R2 | 2 |
| 1d | 2 Gew.-% D1 + 2 Gew.-% A | 3 |

### 2. Desinfektionsversuche

Die Desinfektionswirkung des erfindungsgemäßen Verfahrens wurde gemäß den "Richtlinien für die Prüfung und Bewertung chemischer Desinfektionsverfahren" der Deutschen Gesellschaft für Hygiene und Mikrobiologie (DGHM) im sogenannten quantitativen Suspensionsversuch ermittelt. In diesem Versuch werden 10 ml der zu prüfenden Reinigungs- und Desinfektionslösung mit 0,1 ml von standardisierten Keimsuspensionen gut vermischt. Nach 5, 30 und 60 Minuten werden Proben von jeweils 1 ml entnommen und nach Zusatz einer Inaktivierungsflüssigkeit und weiterer Verdünnung auf Agarplatten ausgestrichen. Zur Kontrolle wird ein gleicher Versuch mit Wasser anstelle der Desinfektionslösung durchgeführt. Nach Bebrütung bei 37 C werden die auf den Agarplatten entstandenen Kolonien ausgezählt. Die Desinfektionswirkung wird in Form eines Faktors wiedergegeben, um den sich die Anzahl der Kolonienbildenden Einheiten (KBE) durch die Einwirkung der Desinfektionslösung in der angegebenen Zeit gegenüber der Behandlung mit Wasser verringert hat. Die Einzelheiten der Versuchsdurchführung sind im Zentralblatt für Bakteriologie, Mikrobiologie und Hygiene 1. Abt. Originale B, Vol. 172, Seite 535 ff, 538 (1980/81) veröffentlicht.

In den folgenden Tabellen sind die als Reduktionsfaktoren ermittelten Ergebnisse in logarithmischer Form aufgeführt. Bei den unter der Bezeichnung Kontrolle wiedergegebenen Zahlenwerten handelt es sich um die Zahlen der überlebenden Keime, wenn anstelle der Desinfektionslösung Wasser eingesetzt wurde.

### 2.1 Prüfung der desinfizierenden Wirkung der Reinigungs- und Desinfektionslösung unmittelbar nach dem Ansetzen und nach Lagerung

Unter Verwendung des Desinfektionskonzentrats D1 sowie des Reinigungskonzentrats R2 und des alkalischen Aktivatorkonzentrats A (zum Vergleich) wurden Reinigungs- und Desinfektionslösungen hergestellt und im oben angegebenen quantitativen Suspensionstest auf ihre desinfizierende Wirkung gegenüber drei Keimen geprüft. Tabelle 2 gibt die Ergebnisse mit den unter Verwendung von 0,5 % Desinfektionskonzentrat hergestellten Lösungen unmittelbar nach Herstellung der Lösungen an; Tabelle 3 zeigt die Ergebnisse mit diesen Lösungen nach 24stündiger Lagerung der Lösungen bei Raumtemperatur. In den Tabellen 4, 5 und 6 sind Ergebnisse mit Reinigungs- und Desinfektionslösungen wiedergegeben, die 2 Gew.-% Desinfektionskonzentrat D1 bzw. 1,5 Gew.-% Desinfektionskonzentrat D2 enthalten und mit und ohne Zusatz von Reinigungskonzentrat R2 hergestellt wurden. Wiedergegeben sind in Tabelle 4 die Desinfektionswerte von Lösungen, die nach der Herstellung zunächst einen Tag gelagert worden waren. Die Tabellen 5 und 6 enthalten Ergebnisse mit 7 bzw. 14 Tage lang gelagerten Reinigungs- und Desinfektionslösungen.

**Tabelle 2:**

| **Desinfektionswirkung mit frischen Lösungen** | | | | |
|---|---|---|---|---|
| **Reinigungs- und Desinfektionslösung** | | **Reduktionsfaktoren nach Einwirkungszeiten** | | |
| **Ausgangskonzentrate** | **Einsatzkonzentration** | **5 Min.** | **30 Min.** | **60 Min.** |
| **Staphylococcus aureus** | | | | |
| D1 | 0,5 % | >5,71 | >5,86 | >5,74 |
| D1 + A | 0,5 % + 1 % | >5,71 | >5,86 | >5,74 |
| D1 + R2 | 0,5 % + 1 % | >5,71 | >5,86 | >5,74 |
| **Kontrolle** | | **6,71** | **6,86** | **6,74** |

| **Pseudomonas aeruginosa** | | | | |
|---|---|---|---|---|
| D1 | 0,5 % | 3,77 | >5,71 | >5,70 |
| D1 + A | 0,5% + 1% | n.a. | 3,53 | >5,70 |
| D1 + R2 | 0,5 % + 1 % | 3,76 | >5,71 | >5,70 |
| **Kontrolle** | | **6,30** | **6,71** | **6,70** |

| **Candida albicans** | | | | |
|---|---|---|---|---|
| D1 | 0,5 % | 3,73 | >4,05 | >4,21 |
| D1 + A | 0,5 % + 1 % | n.a. | 3,40 | >4,21 |
| D1 + R2 | 0,5 % + 1 % | 3,69 | >4,05 | >4,21 |
| **Kontrolle** | | **4,96** | **5,05** | **5,21** |

**Tabelle 3:**

| **Desinfektionswirkung mit 24 h gelagerten Lösungen** | | | | |
|---|---|---|---|---|
| **Reinigungs- und Desinfektionslösung** | | **Reduktionsfaktoren nach Einwirkungszeiten** | | |
| **Ausgangskonzentrate** | **Einsatzkonzentration** | **5 Min.** | **30 Min.** | **60 Min.** |
| **Staphylococcus aureus** | | | | |
| D1 | 0,5 % | 2,25 | >5,87 | >6,04 |
| D1 + A | 0,5 % + 1 % | 5,44 | >5,87 | >6,04 |
| D1 + R2 | 0,5 % + 1 % | >5,89 | >5,87 | >6,04 |
| **Kontrolle** | | **6,89** | **6,87** | **7,04** |

| **Pseudomonas aeruginosa** | | | | |
|---|---|---|---|---|
| D1 | 0,5 % | 2,75 | >5,80 | >5,79 |
| D1 + A | 0,5 % + 1 % | n.a. | n.a. | 3,06 |
| D1 + R2 | 0,5 % + 1 % | n.a. | >5,80 | >5,79 |
| **Kontrolle** | | **6,74** | **6,80** | **6,79** |

| **Candida albicans** | | | | |
|---|---|---|---|---|
| D1 | 0,5 % | 2,79 | >4,22 | >4,26 |
| D1 + A | 0,5 % + 1 % | 0,44 | 3,69 | >4,26 |
| D1 + R2 | 0,5 % + 1 % | 1,08 | >4,22 | >4,26 |
| **Kontrolle** | | **5,25** | **5,22** | **5,26** |

**Tabelle 4:**

| **Desinfektionswirkung mit 24 h gelagerten Lösungen** | | | | |
|---|---|---|---|---|
| **Reinigungs- und Desinfektionslösung** | | **Reduktionsfaktoren nach Einwirkungszeiten** | | |
| **Ausgangskonzentrate** | **Einsatzkonzentration** | **5 Min.** | **30 Min.** | **60 Min.** |
| **Staphylococcus aureus** | | | | |
| D1 | 2 % | >5,70 | >5,49 | >5,56 |
| D1 + R2 | 2 % + 1 % | >5,70 | >5,49 | >5,56 |
| D2 | 1,5 % | >5,70 | >5,49 | >5,56 |
| D2 + R2 | 1,5 % + 1 % | >5,70 | >5,49 | >5,56 |
| **Kontrolle** | | **6,70** | **6,49** | **6,56** |

| **Pseudomonas aeruginosa** | | | | |
|---|---|---|---|---|
| D1 | 2 % | >5,75 | >5,76 | >5,83 |
| D1 + R2 | 2 % + 1 % | >5,75 | >5,76 | >5,83 |
| D2 | 1,5 % | >5,75 | >5,76 | >5,83 |
| D2 + R2 | 1,5 + 1 % | >5,75 | >5,76 | >5,83 |
| **Kontrolle** | | **6,75** | **6,76** | **6,83** |
| **Ausgangskeimzahlen:** Staphylococcus aureus: 4,2X10⁹ KBE/ml Pseudomonas aeruginosa: 9,3X10⁹ KBE/ml | | | | |

**Tabelle 5:**

| **Desinfektionswirkung mit 7 Tage gelagerten Lösungen** | | | | |
|---|---|---|---|---|
| **Reinigungs- und Desinfektionslösung** | | **Reduktionsfaktoren nach Einwirkungszeiten** | | |
| **Ausgangskonzentrate** | **Einsatzkonzentration** | **5 Min.** | **30 Min.** | **60 Min.** |
| **Staphylococcus aureus** | | | | |
| D1 | 2 % | >5,58 | >5,39 | >5,33 |
| D1 + R2 | 2 % + 1 % | >5,58 | >5,39 | >5,33 |
| D2 | 1,5 % | >5,58 | >5,39 | >5,33 |
| D2 + R2 | 1,5 % + 1 % | >5,58 | >5,39 | >5,33 |
| **Kontrolle** | | **6,58** | **6,39** | **6,33** |

| **Pseudomonas aeruginosa** | | | | |
|---|---|---|---|---|
| D1 | 2 % | >6,03 | >5,78 | >5,84 |
| D1 + R2 | 2 % + 1 % | >6,03 | >5,78 | >5,84 |
| D2 | 1,5 % | >6,03 | >5,78 | >5,84 |
| D2 + R2 | 1,5 + 1 % | >6,03 | >5,78 | >5,84 |
| **Kontrolle** | | **7,03** | **6,78** | **6,84** |
| **Ausgangskeimzahlen:** Staphylococcus aureus: 3,3X10⁹ KBE/ml Pseudomonas aeruginosa: 6,8X10⁹ KBE/ml | | | | |

**Tabelle 6:**

| **Desinfektionswirkung mit 14 Tage gelagerten Lösungen** | | | | |
|---|---|---|---|---|
| **Reinigungs- und Desinfektionslösung** | | **Reduktionsfaktoren nach Einwirkungszeiten** | | |
| **Ausgangskonzentrate** | **Einsatzkonzentration** | **5 Min.** | **30 Min.** | **60 Min.** |
| **Staphylococcus aureus** | | | | |
| D1 | 2 % | >5,70 | >5,69 | >5,62 |
| D1 + R2 | 2 % + 1 % | >5,70 | >5,69 | >5,62 |
| D2 | 1,5 % | >5,70 | >5,69 | >5,62 |
| D2 + R2 | 1,5 % + 1 % | >5,70 | >5,69 | >5,62 |
| **Kontrolle** | | **6,70** | **6,69** | **6,62** |

| **Pseudomonas aeruginosa** | | | | |
|---|---|---|---|---|
| D1 | 2 % | >5,84 | >5,81 | >5,82 |
| D1 + R2 | 2 % + 1 % | >5,84 | >5,81 | >5,82 |
| D2 | 1,5 % | >5,84 | >5,81 | >5,82 |
| D2 + R2 | 1,5 + 1 % | >5,84 | >5,81 | >5,82 |
| **Kontrolle** | | **6,84** | **6,81** | **6,82** |
| **Ausgangskeimzahlen:** Staphylococcus aureus: 5,8X10⁹ KBE/ml Pseudomonas aeruginosa: 2,3X10⁹ KBE/ml | | | | |

Aus den Ergebnissen der Tabellen 2 und 3 wird deutlich, daß die gemäß dem erfindungsgemäßen Verfahren unter Zusatz des Reinigungskonzentrats R2 hergestellten Lösungen gegenüber den Keimen Pseudomonas aeruginosa und Candida albicans vor allem nach Lagerung der Lösung deutlich besser abschneiden, als die unter Verwendung des herkömmlichen Aktivators A hergestellen Lösungen. Aus den Ergebnissen in den Tabellen 4, 5 und 6 wird deutlich, daß die gemäß dem erfindungsgemäßen Verfahren verwendeten Reinigungs- und Desinfektionslösungen selbst nach Lagerung über 14 Tage noch eine nahezu unveränderte Desinfektionswirkung aufweisen.

### 2.2 Wirkung gegenüber Mycobakterium chelonae

Durch den Zusatz der Ethanolamine zur Reinigungs- und Desinfektionslösung verbessert sich die Desinfektionslösung gegenüber dem schwer zu bekämpfenden Keim Mycobakterium chaelonae in besonders überraschender Weise und macht dadurch eine Abtötung dieses Keim in vertretbaren Zeiten möglich.

Die in Tabelle 7 aufgeführten Ergebnisse wurden im quantitativen Suspensionstest gegenüber einer 8 Tage alten Kultur von Mycobakterium chelonae (Praxisisolat) bei verlängerten Einwirkungszeiten von 30, 60, 120 und 240 Minuten bei 20 °C ermittelt. Die verwendete Keimsuspension enthielt 5,8X10⁸ KBE/ml.

**Tabelle 7:**

| **Wirkung gegenüber Mycob. chelonae** | | | | | |
|---|---|---|---|---|---|
| **Reinigungs- und Desinfektionslösung** | | **Reduktionsfaktoren nach Einwirkzeiten** | | | |
| **Ausgangskonzentrate** | **Einsatzkonzentrationen** | **30 Min.** | **60 Min.** | **120 Min.** | **240Min.** |
| D2 | 4 % | 0,24 | 0,74 | 1,04 | 1,97 |
| D2 + R2 | 4 % + 1 % | 1,79 | 2,77 | 3,08 | ≥4,67 |
| D2 + R2 | 4 % + 2 % | 1,57 | 2,69 | 2,92 | ≥4,67 |
| **Kontrolle** | | **5,74** | **5,82** | **5,72** | **5,67** |

### 2.3 Wirkung gegenüber Polioviren

Durch den Zusatz der im erfindungsgemäßen Verfahren verwendeten Ethanolamine zur aldehydischen Desinfektionslösung wird auch die Desinfektionswirkung gegenüber Polioviren in überraschender Weise so verstärkt, daß mit niedrigen Konzentrationen an aldehydischem Wirkstoff in vertretbaren Einwirkungszeiten Reduktionsfaktoren über 4 erreicht werden. Die Prüfung der Viruswirksamkeit erfolgte entsprechend der Richtlinie des Bundesgesundheitsamtes und der Deutschen Vereinigung zur Bekämpfung der Viruskrankheiten (Zbl. Hyg. 189, 554 - 562 (1990)). Die verwendete Virussuspension (Polio-Typ 1, Stamm Mahoney) hatte einen Titer von 10^{10,5} ID50/50 Mikroliter. Tabelle 8 gibt die logarithmischen Reduktionsfaktoren nach 15, 30 und 60 Minuten Einwirkzeit wieder.

**Tabelle 8:**

| **Wirkung gegenüber Polioviren** | | | | |
|---|---|---|---|---|
| **Reinigungs- und Desinfektionslösung** | | **Reduktionsfaktoren nach Einwirkungszeiten** | | |
| **Ausgangskonzentrate** | **Einsatzkonzentration** | **15 Min.** | **30 Min.** | **60 Min.** |
| D1 | 2 % | 1,2 | 2,5 | 3,5 |
| D1 + R2 | 2 % + 1 % | 4 | 4,5 | 5 |
| D1 + R2 | 2%+2% | 2,5 | 4 | 4,5 |
| **Kontrolle** | | **9,5** | **9,5** | **9,5** |

## Patentansprüche

1. Verfahren zur Reinigung und Desinfektion von medizinischen Instrumenten, bei dem die zu behandelnden Instrumente in einer ersten Stufe mit einer wäßrigen Reinigungs- und Desinfektionslösung in Kontakt gebracht werden, die mindestens einen Aldehyd aus der Gruppe der Mono- und Dialdehyde mit 1 bis 8 C-Atomen, mindestens ein Tensid sowie gegebenenfalls weitere Hilfsund Zusatzstoffe enthält und einen pH-Wert im alkalischen Bereich aufweist, in einer zweiten Stufe mit Wasser gespült und dann getrocknet werden, **dadurch gekennzeichnet, daß** die Reinigungs- und Desinfektionslösung ein Alkanolamin aus der Gruppe Monoethanolamin, Diethanolamin, Triethanolamin und deren Mischungen enthält.

2. Verfahren nach Anspruch 1, **dadurch gekennzeichnet, daß** die Reinigungsund Desinfektionslösung Diethanolamin enthält.

3. Verfahren nach einem der Ansprüche 1 oder 2, **dadurch gekennzeichnet, daß** die Reinigungs- und Desinfektionslösung weiterhin wenigstens einen Komplexbildner, vorzugsweise aus der Gruppe Nitrilotriessigsäure, Phosphonobutantricarbonsäure und deren lösliche Salze, enthält.

4. Verfahren nach einem der Ansprüche 1 bis 3, **dadurch gekennzeichnet, daß** die Einwirkungszeit der Reinigungs- und Desinfektionslösung auf die medizinischen Instrumente 15 bis 60 Minuten bei Raumtemperatur beträgt.

5. Verfahren nach einem der Ansprüche 1 bis 4, **dadurch gekennzeichnet, daß** die Reinigungs- und Desinfektionslösung einen pH-Wert zwischen 7 und 10, vorzugsweise zwischen 8 und 9,5 aufweist.

6. Verfahren nach einem der Ansprüche 1 bis 5, **dadurch gekennzeichnet, daß** die Reinigungs- und Desinfektionslösung, die auf die medizinischen Instrumente einwirkt, folgende Konzentrationen an Wirkstoffen aufweist:
0,05 - 0,8 Gew.-% Aldehyd
0,05 - 0,5 Gew.-% Tensid
0,05 - 0,3 Gew.-% Alkanolamin
0,02 - 0,3 Gew.-% Komplexbildner

7. Verfahren nach einem der Ansprüche 1 bis 6, **dadurch gekennzeichnet, daß** die Reinigungs- und Desinfektionslösung aus zwei Wirkstoffkonzentraten durch Verdünnen mit Wasser auf die gewünschte Konzentration hergestellt wird, wobei das eine Konzentrat die aldehydischen Wirkstoffe enthält und vorzugsweise einen schwach sauren pH-Wert aufweist, während das zweite Konzentrat Alkanolamin enthält und Tensid sowohl in dem einen als auch in dem anderen Konzentrat enthalten sein kann.

8. Verfahren nach Anspruch 7, wobei das Aldehyd enthaltende Konzentrat folgende Zusammensetzung aufweist:
0,5 - 40 Gew.-%,vorzugsweise 10 - 30 Gew.-% Aldehyd,
0,5 - 20 Gew.-%, vorzugsweise 1 - 10 Gew.-% Tensid
0,1 - 10 Gew.-%, vorzugsweise 0,5 - 5 Gew.-% Komplexbildner
zu 100 Gew.-% Hilfs- und Zusatzstoffe sowie Wasser;
und das Alkanolamin enthaltende Konzentrat folgende Zusammensetzung aufweist:
1 - 20 Gew.-%, vorzugsweise 5 - 15 Gew.-% Alkanolamin
1 - 20 Gew.-%, vorzugsweise 5 - 15 Gew.-% Tensid
1 - 20 Gew.-%, vorzugsweise 5 - 15 Gew.-% Komplexbildner
zu 100 Gew.-% Hilfs- und Zusatzstoffe sowie Wasser.

9. Verfahren nach einem der Ansprüche 1 bis 8, **dadurch gekennzeichnet, daß** in der Reinigungs- und Desinfektionslösung ausschließlich aldehydische Wirkstoffe aus der Gruppe der aliphatischen Mono- und Dialdehyde mit 1 bis 6 C-Atomen, vorzugsweise aus der Gruppe Formaldehyd, Glyoxal, Succindialdehyd, Glutardialdehyd und deren Mischungen verwendet werden.

10. Verfahren nach einem der Ansprüche 1 bis 9, **dadurch gekennzeichnet, daß** in der Reinigungs- und Desinfektionslösung ausschließlich Tenside vom Typ der nichtionischen Tenside, der anionischen Tenside und der zwitterionischen Tesinde, insbesondere allein nichtionische Tenside verwendet werden.

## Claims

1. A process for cleaning and disinfecting medical instruments in which the instruments to be treated are contacted in a first stage with an aqueous cleaning and disinfecting solution which contains at least one aldehyde from the group of C₁₋₈ mono- and dialdehydes, at least one surfactant and optionally other auxiliaries and additives and which has a pH value in the alkaline range and are then rinsed with water and dried in a second stage, **characterized in that** the cleaning and disinfecting solution contains an alkanolamine from. the group consisting of monoethanolamine, diethanolamine, triethanolamine and mixtures thereof.

2. A process as claimed in claim 1, **characterized in that** the cleaning and disinfecting solution contains diethanolamine.

3. A process as claimed in claim 1 or 2, **characterized in that** the cleaning and disinfecting solution additionally contains at least one complexing agent, preferably from the group consisting of nitrilotriacetic acid, phosphonobutanetricarboxylic acid and soluble salts thereof.

4. A process as claimed in any of claims 1 to 3, **characterized in that** the contact time of the cleaning and disinfecting solution with the medical instruments is 15 to 60 minutes at room temperature.

5. A process as claimed in any of claims 1 to 4, **characterized in that** the cleaning and disinfecting solution has a pH value of 7 to 10 and preferably in the range from 8 to 9.5.

6. A process as claimed in any of claims 1 to 5, **characterized in that** the cleaning and disinfecting solution with which the medical instruments are contacted has the following concentrations of active ingredients:
0.05 to 0.8% by weight aldehyde
0.05 to 0.5% by weight surfactant
0.05 to 0.3% by weight alkanolamine
0.02 to 0.3% by weight complexing agent.

7. A process as claimed in any of claims 1 to 6, **characterized in that** the cleaning and disinfecting solution is prepared from two active-substance concentrates by dilution with water to the required concentration, one concentrate containing the aldehydic active substances and preferably having a mildly acidic pH value and the second concentrate containing alkanolamine and surfactant optionally being present both in one and in the other concentrate.

8. A process as claimed in claim 7, **characterized in that** the aldehyde-containing concentrate has the following composition:
0.5 to 40% by weight, preferably 10 to 30% by weight aldehyde,
0.5 to 20% by weight, preferably 1 to 10% by weight surfactant,
0.1 to 10% by weight, preferably 0.5 to 5% by weight complexing agent and
to 100% by weight auxiliaries and additives and water;
and the alkanolamine-containing concentrate has the following composition:
1 to 20% by weight, preferably 5 to 15% by weight alkanolamine,
1 to 20% by weight, preferably 5 to 15% by weight surfactant,
1 to 20% by weight, preferably 5 to 15% by weight complexing agent and
to 100% by weight auxiliaries and additives and water.

9. A process as claimed in any of claims 1 to 8, **characterized in that** the cleaning and disinfecting solution contains only aldehydic active substances from the group of aliphatic mono- and dialdehydes containing 1 to 6 carbon atoms, preferably from the group consisting of formaldehyde, glyoxal, succindialdehyde, glutardialdehyde and mixtures thereof.

10. A process as claimed in any of claims 1 to 9, **characterized in that** only surfactants of the nonionic, anionic and zwitterionic surfactant type, more particularly only nonionic surfactants, are used in the cleaning and disinfecting solution.

## Revendications

1. Procédé de nettoyage et de désinfection d'instruments médicaux, dans lequel les instruments à traiter sont, dans une première étape, mis en contact avec une solution aqueuse de nettoyage et de désinfection qui contient au moins un aldéhyde sélectionné parmi le groupe des mono et dialdéhydes avec 1 à 8 atomes de carbone, au moins un tensioactif ainsi que le cas échéant d'autres adjuvants et additifs, et présente un pH dans le domaine alcalin, sont, dans une deuxième étape, rincés avec de l'eau puis séchés,
**caractérisé en ce que** la solution de nettoyage et de désinfection contient une alcanolamine sélectionnée parmi le groupe des monoéthanolamine, diéthanolamine, triéthanolamine et leurs mélanges.

2. Procédé selon la revendication 1, **caractérisé en ce que** la solution de nettoyage et de désinfection contient de la diéthanolamine.

3. Procédé selon l'une des revendications 1 ou 2, **caractérisé en ce que** la solution de nettoyage et de désinfection contient en outre au moins un agent complexant, de préférence sélectionné parmi le groupe des acide nitrilotriacétique, acide phosphonobutanetricarboxylique et leurs sels solubles.

4. Procédé selon l'une des revendications 1 à 3, **caractérisé en ce que** le temps d'action de la solution de nettoyage et de désinfection sur les instruments médicaux s'élève à température ambiante de 15 à 60 minutes.

5. Procédé selon l'une des revendications 1 à 4, **caractérisé en ce que** la solution de nettoyage et de désinfection présente un pH entre 7 et 10, de préférence entre 8 et 9,5.

6. Procédé selon l'une des revendications 1 à 5, **caractérisé en ce que** la solution de nettoyage et de désinfection qui agit sur les instruments médicaux, présente les concentrations en substances actives suivantes :
0,05-0,8% en poids d'aldéhyde;
0,05-0,5% en poids de tensioactif;
0,05-0,3% en poids d'alcanolamine, et
0,02-0,3% en poids d'agent complexant.

7. Procédé selon l'une des revendications 1 à 6, **caractérisé en ce que** la solution de nettoyage et de désinfection constituée de deux concentrés de substances actives est fabriquée par dilution avec de l'eau à la concentration souhaitée, le premier concentré contenant les substances actives aldéhyde et présentant de préférence un pH faiblement actif, tandis que le deuxième concentré contient de l'alcanolamine et qu'un tensioactif peut être contenu autant dans le premier concentré que dans le deuxième.

8. Procédé selon la revendication 7, le concentré contenant l'aldéhyde présentant la composition suivante :
0,5-40% en poids, de préférence 10-30% en poids d'aldéhyde;
0,5-20% en poids, de préférence 1-10% en poids de tensioactif;
0,1-10% en poids, de préférence 0,5-5% en poids d'agent complexant, et
jusque 100% en poids d'adjuvants et d'additifs ainsi que de l'eau;
et le concentré contenant l'alcanolamine présentant la composition suivante :
1-20% en poids, de préférence 5-15% en poids d'alcanolamine;
1-20% en poids, de préférence 5-15% en poids de tensioactif;
1-20% en poids, de préférence 5-15% en poids d'agent complexant, et
jusque 100% en poids d'adjuvants et d'additifs, ainsi que de l'eau.

9. Procédé selon l'une des revendications 1 à 8, **caractérisé en ce que**, dans la solution de nettoyage et de désinfection, on utilise exclusivement des substances aldéhyde sélectionnées parmi le groupe des mono et dialdéhydes aliphatiques avec 1 à 6 atomes de carbone, de préférence sélectionnées parmi le groupe des formaldéhyde, glyoxal, dialdéhyde succinique, dialdéhyde glutarique et leurs mélanges.

10. Procédé selon l'une des revendications 1 à 9, **caractérisé en ce que**, dans la solution de nettoyage et de désinfection, on utilise exclusivement des tensioactifs du type des tensioactifs non ioniques, des tensioactifs anioniques et des tensioactifs ioniques mixtes, en particulier uniquement des tensioactifs non ioniques.
